Europäisches Patentamt

European Patent Office

Office européen des brevets

(19)

(11) Publication number : **0 365 480 B1**

(12) **EUROPEAN PATENT SPECIFICATION**

(45) Date of publication of patent specification :
**19.08.92 Bulletin 92/34**

(51) Int. Cl.⁵ : **A61K 31/195, A61K 9/20**

(21) Application number : **89810772.7**

(22) Date of filing : **10.10.89**

(54) **Dispersible formulation.**

The file contains technical information
submitted after the application was filed and
not included in this specification

(30) Priority : **18.10.88 GB 8824392**

(43) Date of publication of application :
**25.04.90 Bulletin 90/17**

(45) Publication of the grant of the patent :
**19.08.92 Bulletin 92/34**

(84) Designated Contracting States :
**AT BE CH DE ES FR GB GR IT LI LU NL SE**

(56) References cited :
**EP-A- 0 255 002**
**EP-A- 0 324 981**

(73) Proprietor : **CIBA-GEIGY AG**
**Klybeckstrasse 141**
**CH-4002 Basel (CH)**

(72) Inventor : **Murphy, Lorraine Mary**
**4 Nightingale Road**
**Horsham West Sussex RH12 2NW (GB)**
Inventor : **Matthews, Graham Paul**
**23 Fenhurst Close Hills Farm Lane**
**Horsham West Sussec RH12 1UX (GB)**

(74) Representative : **Sharman, Thomas et al**
**CIBA-GEIGY PLC. Patent Department, Central**
**Research, Hulley Road**
**Macclesfield, Cheshire SK10 2NX (GB)**

EP 0 365 480 B1

Jouve, 18, rue Saint-Denis, 75001 PARIS

## Description

The present invention relates to a dispersable solid dry formulation containing diclofenac.

Diclofenac is an effective analgesic and antiarthritic agent. It is available, inter alia, as enteric coated tablets and sustained release tablets containing diclofenac sodium, and also as sugar coated tablets of diclofenac potassium.

EP-A-255002 describes galenic formulations with programmed release, immediate and delayed comprising a mixture of a granulate having immediate release and a granulate having a controlled release. Diclofenac is mentioned among other active ingredients as a possible component. No specific formulation containing diclofenac either as the free acid or as a salt is described. The formulations are in the form of tablets, capsules, sugar coated tablets and granulates for suspension or for solution in suitable liquid media, although it is not clear how the last mentioned form is possible in view of the delayed release portion.

Some patients are unable or unwilling to swallow tablets, and for these patients, and others, a tablet which disperses in water or other suitable liquid is advantageous because it is more acceptable. Being swallowed in dispersed or dissolved form, the drug is rapidly effective.

If diclofenac sodium is incorporated in a dispersible tablet it dissolves when the tablet is dispersed in water or other suitable liquid producing a liquid with an undesirable bitter taste. Diclofenac potassium also produces a liquid with a bitter taste.

We have found that this difficulty is overcome if diclofenac is dispersed in finely divided form as the free acid rather than as a salt. This has a low solubility and is virtually tasteless.

Accordingly the present invention provides a dispersible solid drug formulation comprising finely divided diclofenac in a particle size diameter of from 4 to 100 μm as the free acid, from 5 to 25% by weight of a disintegrant, a pharmaceutically acceptable diluent and optionally a wetting agent and/or lubricant.

As disintegrant there may be used compounds such as microcrystalline cellulose, starches and starch derivatives. Preferably a compound known as a superdisintegrant is used, such as croscarmellose, crospovidone and sodium starch glycollate. In some instances it is advantages to use a combination of disintegrants.

The amount of disintegrant, or mixture thereof, is from 5 to 25 %, preferably from 5 to 15 %. We prefer to use higher concentrations of disintegrant than is normally used in a conventional (i.e. non-dispersible) formulation.

The formulations of the invention also contain at least one diluent in order to give sufficient material to tablet and facilitate the compression process used to make tablets. Suitable diluents include microcrystalline cellulose, calcium hydrogen phosphate, and lactose. The function of the diluent may be performed by other components, especially disintegrants.

The formulation of the invention may also contain wetting agents to improve the disintegration and/or dispersion. Suitable wetting agents include dioctyl sodium sulphosuccinate, polysorbates or sodium lauryl sulphate. The amount of wetting agent is usually not more than 0.1 % by weight of the formulation.

The formulation of the invention may also include lubricants, including agents to improve flow. Suitable compounds include fatty acids such as stearic acid, metal stearates such as magnesium stearate, hydrogenated castor oil, talc, and colloidal silicon dioxide. Lubricants may be used in amounts of up to 2 % by weight of the formulation.

Colours, flavours and aromatising agents may also be included in the formulations.

The solid drug formulations may be in the form of a simple mixture of the ingredients which can be filled into sachets that can be emptied into water. Preferably the solid drug formulations are in the form of tablets.

Tablets can be manufactured in several known different ways. In the so-called direct compression process a suitable diluent, for example microcrystalline cellulose, selected grades of calcium hydrogen phosphate, or lactose, is chosen to allow the components to be mixed and tabletted.

In the so-called wet granulation process, most of the components of the formulation, including the diclofenac, diluent and all or part of the disintegrant are formed into granules by the addition of a liquid, usually water, and optionally a binding agent. The remaining components such as the remainder of the disintegrant and lubricants are then added and the blend tabletted. If colour and/or flavours are used they may be added at any stage of the process.

The invention is illustrated by the following Examples.

### Example 1:

Diclofenac free acid, lactose and an aliquot of sodium croscarmellose are granulated with an aqueous solution of hydroxypropyl methylcellulose 3 mPa.s and sodium lauryl sulphate in a fluid bed granulator. The granules are dried and then blended with the remaining excipients and compressed into tablets having the fol-

lowing composition.

|                                       | Quantity mg/tablet |
|---------------------------------------|--------------------|
| DICLOFENAC                            | 46.5               |
| Microcrystalline Cellulose            | 100.0              |
| Lactose BP                            | 100.0              |
| Sodium Croscarmellose                 | 21.0               |
| Hydroxypropylmethylcellulose 3 cps    | 1.82               |
| Hydrogenated Castor Oil               | 1.5                |
| Purified Talc                         | 1.5                |
| Sodium Lauryl Sulphate                | 0.045              |
| Total weight of Tablet                | 272.365 mg         |

Example 2:

Tablets are made by the method of Example 1 except that calcium hydrogen phosphate is used in place of the microcrystalline cellulose and lactose. The tablets have the following composition.

|                                       | Quantity mg/tablet |
|---------------------------------------|--------------------|
| DICLOFENAC                            | 46.5               |
| Calcium Hydrogen Phosphate            | 200.0              |
| Sodium Croscarmellose                 | 18.0               |
| Hydroxypropylmethylcellulose 3 cps    | 1.82               |
| Hydrogenated Castor Oil               | 1.5                |
| Purified Talc                         | 1.5                |
| Sodium Lauryl Sulphate                | 0.045              |
| Total weight of Tablet                | 269.365 mg         |

Example 3:

Diclofenac free acid is dry blended with microcrystalline cellulose, sodium crosscarmellose and colouring material. The mass is then wet granulated with water. The granules are then blended with the remainder of the excipients and compressed into tablets having the following composition.

|                              | Quantity mg/tablet |
|------------------------------|:-----:|
| DICLOFENAC                   | 46.5  |
| Microcrystalline cellulose   | 158.5 |
| F.D. & C. Red No. 3          | 0.3   |
| F.D. & C. Red No. 3/Al Lake  | 1.3   |
| Blackcurrant Flavour         | 30.0  |
| Sodium saccharin BP          | 2.5   |
| Sodium croscarmellose        | 14.5  |
| Sodium starch glycollate     | 29.0  |
| Hydrogenated Castor Oil      | 1.5   |
| Purified Talc                | 1.5   |
| Colloidal silicon dioxide    | 4.4   |
| Total weight of Tablet       | 290 mg |

Example 4:

Diclofenac free acid is blended with all the excipients other than the lubricant. The mixture is then blended with the lubricant and compressed into tablets having the following composition.

|                              | Quantity mg/tablet |
|------------------------------|:-----:|
| DICLOFENAC                   | 46.5  |
| Microcrystalline cellulose   | 180.2 |
| F.D. & C. Red No. 3          | 0.3   |
| F.D. & C. Red No. 3/Al Lake  | 1.3   |
| Blackcurrant Flavour         | 30.0  |
| Sodium saccharin             | 2.5   |
| Purified Talc                | 3.0   |
| Hydrogenated Castor Oil      | 3.0   |
| Sodium croscarmellose        | 23.2  |
| Total weight of Tablet       | 290.0 mg |

Example 5:

Example 4 is repeated to produce tablets having the following composition.

4

|  | Quantity mg/tablet |
| --- | --- |
| DICLOFENAC | 46.5 |
| Microcrystalline cellulose | 180.2 |
| F.D. & C. Red No. 3 | 0.3 |
| F.D. & C. Red No. 3/Al Lake | 1.3 |
| Blackcurrant Flavour | 30.0 |
| Sodium saccharin | 2.5 |
| Sodium croscarmellose | 23.2 |
| Magnesium Stearate | 6.0 |
| Total weight of Tablet | 290.0 mg |

## Claims

1. A dispersible solid drug formulation comprising finely divided diclofenac in a particle size diameter of from 4 to 100 µm as the free acid, from 5 to 25 % by weight of a disintegrant, a pharmaceutically acceptable diluent and optionally a wetting agent and/or lubricant.

2. A formulation as claimed in claim 1 in which the disintegrant is microcrystalline cellulose, croscarmellose, crospovidone, sodium starch glycollate, starch or a derivative thereof, or a mixture of two or more disintegrants.

3. A formulation as claimed in any preceding claim in which the amount of disintegrant is from 5 to 15 % by weight.

4. A formulation as claimed in any preceding claim in which the diluent is microcrystalline cellulose, calcium hydrogen phosphate and/or lactose.

5. A formulation as claimed in any preceding claim which also contains up to 0.1 % by weight of a wetting agent.

6. A formulation as claimed in any preceding claim which also contains up to 2 % by weight of lubricant.

7. A formulation as claimed in any preceding claim which is in the form of a tablet.

8. A process for the preparation of a dispersible solid drug formulation as defined in claims 1 to 6, which process comprises formulating by mixing finely divided diclofenac as the free acid, in an amount as given in claim 1 or 3 a

9. A process for the preparation of a dispersible solid drug formulation in form of a tablet as defined in claims 1 to 6, which comprises formulating by mixing finely divided diclofenac, in an amount as given in claim 1 or 3 a disintegrant, a pharmaceutically acceptable diluent and optionally a wetting agent and/or lubricant and compressing to tablets.

## Patentansprüche

1. Dispergierbare feste Arzneimittelformulierung, die fein verteiltes Diclofenac als freie Säure in einem Teilchengrößendurchmesser von 4 bis 100 µm, 5 bis 25 Gew.% eines Sprengmittels, ein pharmazeutisch annehmbares Streckmittel, sowie gegebenenfalls ein Netzmittel und/oder Schmiermittel enthält.

2. Formulierung nach Anspruch 1, worin es sich bei dem Sprengmittel um mikrokristalline Zellulose, Croscarmellose, Crospovidon, Natriumstärkeglykolat, Stärke oder ein Derivat davon oder eine Mischung von zwei oder mehr Sprengmitteln handelt.

3. Formulierung nach einem der vorhergehenden Ansprüche, worin die Menge an Sprengmittel 5 bis 15 Gew.% beträgt.

4. Formulierung nach einem der vorhergehenden Ansprüche, worin es sich bei dem Streckmittel um mikrokristalline Zellulose, Kalziumhydrogenphosphat und/oder Laktose handelt.

5. Formulierung nach einem der vorhergehenden Ansprüche, die ebenfalls bis zu 0,1 Gew.% eines Netzmittels enthält.

6. Formulierung nach einem der vorhergehenden Ansprüche, die ebenfalls bis zu 2 Gew.% eines Schmiermittels enthält.

7. Formulierung nach einem der vorhergehenden Ansprüche, die in Form einer Tablette vorliegt.

8. Verfahren zur Herstellung einer dispergierbaren, festen Arzneimittelformulierung wie in Ansprüchen 1 bis 6 definiert, das darin besteht, daß man fein verteiltes Diclofenac als freie Säure in einer in Anspruch 1 oder 3 angegebenen Menge unter Vermischen formuliert.

9. Verfahren zur Herstellung einer dispergierbaren, festen Arzneimittelformulierung in Form von Tabletten wie in Ansprüchen 1 bis 6 definiert, das darin besteht, daß man fein verteiltes Diclofenac als freie Säure in einer in Anspruch 1 oder 3 angegebenen Menge, ein Sprengmittel, ein pharmazeutisch annehmbares Streckmittel und gegebenenfalls ein Netzmittel und/oder ein Schmiermittel unter Vermischen formuliert und zu Tabletten preßt.

## Revendications

1. Une formule de médicament solide dispersable comprenant du diclofénac finement divisé, en particules de 4 à 100 µm de diamètre, à l'état d'acide libre, avec de 5 à 25 % en poids d'un agent de désagrégation, un diluant pour usages pharmaceutiques et le cas échéant un agent mouillant et/ou un lubrifiant.

2. Une formule selon la revendication 1 dont l'agent désagrégeant est de la cellulose microcristalline, de la croscarmellose, de la crospovidone, du glycolate d'amidon sodique, de l'amidon ou un dérivé d'amidon ou un mélange de deux ou plus de ces matières.

3. Une formule selon la revendication 1 ou 2 dont la proportion de l'agent désagrégeant est de 5 à 15 % en poids.

4. Une formule selon l'une quelconque des revendications précédentes dont le diluant est de la cellulose microcristalline, de l'hydrogénophosphate de calcium et/ou du lactose.

5. Une formule selon l'une quelconque des revendications précédentes qui comprend aussi jusqu'à 0,1 % en poids d'un agent mouillant.

6. Une formule selon l'une quelconque des revendications précédentes qui comprend aussi jusqu'à 2 % en poids d'un lubrifiant.

7. Une formule selon l'une quelconque des revendications précédentes, présentée en comprimés.

8. Un procédé de préparation d'une formule de médicament solide dispersable telle que définie aux revendications 1 à 6, procédé selon lequel on mélange du diclofénac à l'état d'acide libre finement divisé avec une proportion telle qu'indiquée à la revendication 1 ou 3 d'un agent de désagrégation, un diluant pour usages pharmaceutiques et le cas échéant un agent mouillant et/ou un lubrifiant.

9. Un procédé de préparation d'une formule de médicament solide dispersable en comprimés, telle que définie aux revendications 1 à 6, procédé selon lequel on mélange du diclofénac à l'état d'acide libre finement divisé avec une proportion telle qu'indiquée à la revendication 1 ou 3 d'un agent désagrégeant, un diluant pour usages pharmaceutiques et le cas échéant un agent mouillant et/ou un lubrifiant, et on comprime le mélange pour en faire des comprimés.